# EUROPÄISCHE PATENTSCHRIFT

(11) **EP 0 583 687 B1**
(45) Veröffentlichungstag und Bekanntmachung des Hinweises auf die Patenterteilung: **13.01.1999**
(21) Anmeldenummer: 93112492.9
(22) Anmeldetag: 04.08.1993
(51) Int. Cl.: C12P 7/22, C12P 7/40

(54) **Verfahren zur Herstellung substituierter Methoxyphenole und dafür geeignete Mikroorganismen**
Method for the preparation of substituted methoxyphenols and microorganisms therefore
Procédé de production de méthoxyphénols substitués et microorganismes pour ce procédé

(30) Priorität: 17.08.1992 DE 4227076
(43) Veröffentlichungstag der Anmeldung: 23.02.1994
(73) Patentinhaber: HAARMANN & REIMER GMBH, D-37601 Holzminden (DE)
(72) Erfinder: Hopp, Rudolf, Dr., D-37603 Holzminden (DE); Rabenhorst, Jürgen, Dr., D-37671 Höxter (DE)
(74) Vertreter: Zobel, Manfred, Dr.

(56) Entgegenhaltungen:
- US-A- 3 817 830
- US-A- 5 128 253
- AGRICULTURAL AND BIOLOGICAL CHEMISTRY, Bd.47, November 1983, TOKYO JP Seiten 2639 - 2640 K.TADASA ET AL.
- DATABASE WPI Week 9038, Derwent Publications Ltd., London, GB; AN 90-285861 & JP-A-2 200 192 (HASEGAWA KK) 8. August 1990
- DATABASE WPI Week 9037, Derwent Publications Ltd., London, GB; AN 90-278836 & JP-A-2 195 871 (HASEGAWA KK) 2. August 1990
- CHEMICAL ABSTRACTS, vol. 84, no. 10, 8. März 1976, Columbus, Ohio, US; abstract no. 61523d, H.KAWAKAMI Seite 119 ;Spalte 1 ; & MOKUZAI GAKKAISHI, Bd.21, Nr.5, 1975 Seiten 309 - 314
- DATABASE WPI Week 9340, Derwent Publications Ltd., London, GB; AN 93-316614 & JP-A-5 227 980 (TAKASAGO PERFUMERY CO LTD) 7. September 1993

## Beschreibung

Die Erfindung betrifft ein Verfahren zur Herstellung substituierter o-Methoxyphenole aus Eugenol oder Eugenol enthaltenden Gemischen mit Hilfe von Mikroorganismen und für dieses Verfahren geeignete Bakterien.

Substituierte Methoxyphenole, wie z.B. Coniferylalkohol und Coniferylaldehyd, sind als Aromastoffe verwendbar. Allerdings sind Coniferylalkohol und insbesondere Coniferylaldehyd in größeren Mengen gar nicht verfügbar. Es bestand daher das Bedürfnis nach einem in technischem Umfang wirtschaftlich nutzbaren Verfahren.

Andere Methoxyphenole eignen sich als günstige Zwischenprodukte für die Herstellung verschiedener natürlicher Aromastoffe. So wird beispielsweise die Herstellung von Vanillin u.a. aus Ferulasaure beschrieben (EP-A-453 368). Auch in dem US Patent 5 128 253 wird die Produkt von Vanillin aus Ferulasäure beschrieben. Hier werden verschiedene Mikroorganismen verwendet, die in der Lage sind, Ferulasäure zu verstoffwechseln. Für die Vanillinbildung bedarf es aber der Zugabe wasserlöslicher Sulfhydrylkomponenten wie z.B. DTT (Dithiotreitol). Die erreichten Konzentrationen an Vanillin sind aber in beiden Fällen sehr niedrig. Ferulasäure ist schon als synthetisches Produkt relativ teuer. Die Herstellung natürlicher Aromastoffe erfordert aber den Einsatz natürlicher Ausgangsmaterialien. Natürliche Ferulasäure ist aber bislang nicht erhältlich. Sie kommt lediglich in geringen Konzentrationen in verschiedenen pflanzlichen Materialien vor - meist in gebundener Form (Ester). Eine natürliche Isolierung daraus ist wirtschaftlich nicht sinnvoll. Auch für Vanillinsäure ist keine günstige natürliche Bezugsquelle bekannt.

In der Literatur (K. Tadasa and H. Kayahara, Agric. Biol. Chem, 47, 2629-2640, 1983) wurde schon einmal der Abbau von Eugenol über Coniferylalkohol und Ferulasäure mit einer Pseudomonas sp. beschrieben. Aufgrund der Beschreibung muß es sich um eine andere Art handeln; entscheidend ist aber, daß der Stamm nicht hinterlegt worden ist und damit für eine Nacharbeitung nicht zur Verfügung steht.

Es wurde nun aus einer Bodenprobe aus Ostjava/Indonesien eine neue Pseudomonaden sp. isoliert, die überraschenderweise in der Lage ist, Eugenol zu Ferulasäure, Vanillinsäure, Coniferylalkohol und Coniferylaldehyd umzusetzen. Eugenol ist ein relativ preiswerter Bestandteil von natürlichem Nelkenöl.

Gegenstand der Erfindung ist also die neue Art Pseudomonas sp. nov. mit den bei der Deutschen Sammlung für Mikroorganismen und Zellkulturen GmbH in Braunschweig unter den Nummern DSM 7062 und DSM 7063 hinterlegten Stämmen.

| Taxonomische Beschreibung der Art | |
|---|---|
| Zellform: | Stäbchen |
| Breite (µm) | 0,5 - 0,7 |
| Länge (µm) | 1,2 - 2,5 |
| Beweglichkeit | + |
| Geißeln | polar 1 |
| Gram-Reaktion | negativ |
| Lyse durch 3 % KOH | + |
| Aminopeptidase (Cerny) | + |
| Sporen | - |
| Oxidase | + |
| Catalase | + |
| | |
| Wachstum | |
| anaerob | - |
| 37/41 °C | +/+ |
| pH 5,6 | + |
| MacConkey-Agar | + |
| SS-Agar | + |
| Cetrimid-Agar | + |
| | |
| Pigmente | |
| nicht diffundierend | - |
| diffundierend | - |
| fluoreszierend | - |
| Pyocyanin | - |
| | |
| Gas aus Glucose | - |
| | |
| Säure aus (ASS) | |
| Glucose | + |
| Fructose | + |
| ADH | + |
| ONPG | - |
| VP | - |
| Indol | - |
| NO₂ aus NO₃ | + |
| Phenyldesaminase | - |
| Levan aus Saccharose | - |
| Urease | - |
| | |
| Hydrolyse von | |
| Starke | - |
| Casein | - |
| DNA | - |
| Tween 80 | - |
| Äsculin | - |
| | |
| Wuchsstoffbedarf | - |
| | |
| Substratverwertung | |
| Acetat | + |
| Adipat | + |
| Azelat | + |
| Caprat | + |
| Citrat | + |
| Glycolat | - |
| Lactat | + |
| Lävulinat | + |
| Malat | + |
| Malonat | - |
| Mucat | + |
| Sebacinat | + |
| Suberat | - |
| Phenylacetat | + |
| L-Arabinose | - |
| Fructose | + |
| Glucose | + |
| Mannose | - |
| Xylose | - |
| Galactose | - |
| Trehalose | - |
| Saccharose | - |
| Saccharat | - |
| Mannitol | - |
| Inositol | - |
| Gluconat | + |
| Alanin | + |
| Hydroxybutyrat | + |
| Geraniol | + |
| Benzoylformiat | - |

Einzelne geringfügige Abweichungen, insbesondere im Substratverwertungsspektrum, sind innerhalb einer Art üblich und nicht auszuschließen.

Weiterer Gegenstand der Erfindung ist ein Verfahren zur Herstellung von Verbindungen der Formel 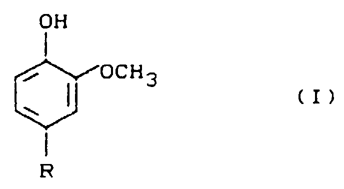 worin
- R: -COOH, -CH=CH-COOH, -CH=CH-CH₂OH oder -CH=CH-CHO bedeutet,
aus Eugenol 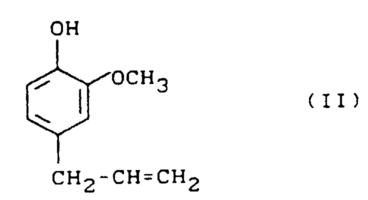 in Gegenwart von Pseudomonas sp. nov. oder deren Enzymen oder von Mikrorganismen mit genetischem Material aus Pseudomonas sp. nov., das die Struktur- und Regulatorgene für die Enzyme kodiert, die in dieser Reaktion wirksam sind.

Eugenol kann auch in Mischung mit anderen Komponenten eingesetzt werden, beispielsweise in Form von Nelkenöl.

Die erfindungsgemäß zu verwendende Art Pseudomonas sp. nov. kann in üblichen Kulturmedien kultiviert werden. Diese Kulturmedien können synthetisch, halbsynthetisch oder komplex sein und können Kohlenstoffquellen, Stickstoffquellen, anorganische Salze und gegebenenfalls Spurenelemente und Vitamine enthalten.

Als Kohlenstoffquellen können z.B. Zucker wie Glucose, Zuckeralkohole wie Glycerin, organische Säuren wie Zitronensäure oder komplexe Gemische wie Malzextrakt, Hefeextrakt, Casein oder Caseinhydrolysat dienen.

Beispiele für geeignete Stickstoffquellen sind anorganische Stickstoffquellen wie Nitrate und Ammoniumsalze und organische Stickstoffquellen wie Hefeextrakt, Sojamehl, Baumwollsaatmehl, Casein, Caseinhydrolysat, Weizengluten und Maisquellwasser.

Als organische Salze können beispielsweise unter anderem Sulfate, Nitrate, Chloride, Carbonate und Phosphate von Natrium, Kalium, Magnesium, Calcium, Zink und Eisen verwendet werden.

Das Substrat kann zu Beginn der Inkubation, während oder nach Abschluß des Wachstums auf einmal oder über einen längeren Zeitraum verteilt zugegeben werden. Die Menge an Eugenol wird dabei vorteilhaft so bemessen, daß die Konzentration der Verbindung in der Kulturbrühe 30 g/l, vorzugsweise 5 g/l nicht überschreitet. Der Verlauf der Oxidation kann durch Bestimmung des Ausgangsmaterials und der Produkte in der Kulturbrühe mittels Hochdruckflüssigkeitschromatographie verfolgt werden. Nachdem die optimale Menge der gewünschten Substanzen entstanden ist, können diese durch bekannte physikalische Verfahren wie Extraktion, Destillation oder Chromatographie aus der Kulturbrühe isoliert und durch weitere Schritte gereinigt werden.

Die Kultivierungstemperatur liegt vorzugsweise im Bereich von 10 bis 48°C, besonders bevorzugt im Bereich von 16 bis 37°C. Der pH-Wert des Mediums beträgt bevorzugt 3 bis 9, insbesondere 4 bis 8. Die Kultivierung kann beispielsweise in geeigneten Schüttelapparaturen oder in Fermentern mit Mischeinrichtung erfolgen. Bei der Kultivierung ist für eine ausreichende Belüftung Sorge zu tragen. Die Kultivierung kann batchweise, halbkontinuierlich oder kontinuierlich durchgeführt werden. Die Kulturdauer bis zum Erreichen einer maximalen Produktmenge liegt zwischen 4 und 240 Stunden. Um die Mikroorganismen vor der toxischen Wirkung des Eugenols zu schützen, kann es vorteilhaft sein, den Kulturmedien Adsorptionsmittel für das Substrat zuzusetzen, z.B. Aktivkohle oder Adsorberharze.

Durch Wahl der geeigneten Prozeßparameter ist es möglich, einzelne der oben erwähnten Stoffe als Hauptkomponenten der Umsetzung zu erhalten.

### Beispiele

### Herstellung der Vorkultur

Ein 500 ml Erlenmeyerkolben wurde mit 100 ml Nutrient Broth gefüllt und anschließend 20 min bei 121°C sterilisiert. Nach dem Abkühlen wurde der Kolben mit einer Impföse mit einer Kultur Pseudomonas sp. nov. DSM 7062 oder DSM 7063 von einer Agarplatte beimpft. Die Kultur wurde auf einer rotierenden Schüttelmaschine bei 27°C und 120 Upm inkubiert. Nach 17 Stunden wurde diese Kultur zur Beimpfung des Produktionsmediums verwendet.

### Beispiel 1

### Produktion im Schüttelkolben

Neun 500 ml Erlenmeyerkolben wurden mit je 100 ml Medium (4 g K₂HPO₄, 1 g NaH₂PO₄, 0,5 g Hefeextrakt, 0,2 g NaCl, 0,2 g MgSO₄ x 7 H₂O, 0,05 g CaCl₂, 1000 ml Wasser, pH 7,25) gefüllt und anschließend 20 min bei 121°C dampfsterilisiert. Nach dem Abkühlen wurden die Kolben mit je 5 ml einer Kultur von DSM 7063 angeimpft. Die Kulturen wurden auf einer rotierenden Schüttelmaschine bei 27°C und 120 Upm inkubiert. Gleich nach dem Animpfen wurden 500 ppm Eugenol zugegeben, sowie nach 4,5 Stunden nochmals 500 ppm, nach 9, 24 und 32,75 Stunden jeweils weitere 1000 ppm. Nach 54 Stunden wurde die Fermentation abgebrochen, die Kolben autoklaviert, die Kulturbrühe der Kolben vereinigt und die Zellmasse abgetrennt. Der Gehalt an Ferulasäure in der Kulturbrühe lag gemäß HPLC-Analysen bei 1280 ppm entsprechend einer Umsetzung von 32 % der Theorie. Der Gehalt von Coniferylalkohol lag bei 225 ppm und der Gehalt an Vanillinsäure bei 131 ppm.

### Isolierung der Ferulasäure

810 ml des in Beispiel 1 gewonnenen Kulturfiltrats wurden mit 1 N HCl auf pH 4,0 eingestellt und mit dem gleichen Volumen Ethylacetat einmal extrahiert. Die organische Phase wurde getrennt und das Lösungsmittel bis zur Trockene am Rotationsverdampfer abgezogen. Es wurde ein Niederschlag von 1,31 g erhalten, der zu 71 % Ferulasäure enthielt. Dies entspricht einer Wiedergewinnung von 90 %.

### Beispiel 2

### Produktion im 10 l Fermenter

10 l Kulturmedium (100 g Malzextrakt, 40 g Glucose, 20 g Hefeextrakt, 10 000 ml Wasser, pH 7,3) wurden in einem Fermenter sterilisiert und nach Abkühlen mit 0,5 l einer Vorkultur DSM 7063 angeimpft. Die Kulturbedingungen waren: 32°C, 200 Upm, 8 l Luft/min. Gleich nach dem Animpfen wurden 1000 ppm Eugenol zugegeben, nach 7 Stunden 500 ppm, sowie nach 10,5, 16, 22, 25 und 28 Stunden nochmals je 1000 ppm. Zusätzlich wurde die Luft nach 25 Stunden auf 4 l Luft/min reduziert.

Nach 32,5 Stunden wurde die Fermentation abgebrochen. Die Konzentration der einzelnen Komponenten lag bei:

| | | |
|---|---|---|
| Ferulasäure | 2365 ppm | (31 % der Theorie) |
| Coniferylalkohol | 1458 ppm | (20 % der Theorie) |
| Vanillinsäure | 1314 ppm | (20 % der Theorie) |
| Coniferylaldehyd | 11 ppm | |

Am Ende des Verfahrens lagen noch 270 ppm (4 %) nicht umgesetztes Eugenol vor.

### Beispiel 3

### Produktion von Vanillinsäure im 10 l Fermenter

10 l Kulturmedium (KH₂PO₄ 16 g; Na₂HPO₄ 30 g; NH₄Cl 5,3 g; MgSO₄ x 7 H₂O 1 g; CaCl₂ x 2 H₂O 0,7 g; Hefeextrakt 10 g ad 10 L deion. H₂O; pH 7,0) wurden in einem Fermenter sterilisiert und nach Abkühlen wie in Beispiel 2 angeimpft. Die Kulturbedingungen waren: 27°C, 200 Upm, 4 l Luft/min. Gleich nach dem Animpfen wurden 1000 ppm Eugenol zugegeben, sowie nach 12, 20, 23,5, 28,5 und 36 Stunden nochmals je 1000 ppm.

Nach 99 Stunden wurde die Fermentation abgebrochen. Die Konzentration an Vanillinsäure lag bei 3,25 g/l, entsprechend einer Umsetzung von 52,9 % der Theorie.

### Beispiel 4

### Produktion von Coniferylalkohol

10 l Nutrient Broth wurden in einem Fermenter sterilisiert und nach dem Abkühlen mit einer 24 Stunden alten Vorkultur von DSM 7063 angeimpft. Die Kulturbedingungen waren 27°C, 400 Upm, 4 l Luft/min. Nach 15 Stunden wurde diese Kultur steril in einem Fermenter mit 190 l Medium entsprechend Beispiel 1 übergeführt und bei 27°C, 200 Upm und 40 l Luft/min. kultiviert. Zu Beginn der Fermentation wurden 1000 ppm Eugenol zugegeben, sowie nach 7,5 Stunden 250 ppm, nach 14 Stunden 1000 ppm, nach 14,5 Stunden und nach 23,5 Stunden 250 ppm, sowie ab der 25. Stunde während der nächsten 6 Stunden 1405 ppm und ab der 32. Stunde während der nächsten 15,5 Stunden 2590 ppm. Insgesamt wurden 6745 ppm Eugenol über einen Zeitraum von 47,5 Stunden zugegeben.

Die Ausbeute an Coniferylalkohol lag bei 3226 ppm. Dies sind 43,5 % der Theorie. Daneben wurden noch 1738 ppm (25 % der Theorie) Ferulasäure, sowie 12 ppm Coniferylaldehyd und 8 ppm Vanillinsäure erhalten. Am Ende des Verfahrens lagen noch 496 ppm (7,3 %) nicht umgesetztes Eugenol vor.

### Beispiel 5

### Produktion von Ferulasäure

10 l Kulturmedium (40 g K₂HPO₄, 10 g NaH₂PO₄, 5 g Caseinhydrolysat, 2 g NaCl, 2 g MgSO₄ x 7 H₂O, ad 10 l Wasser, pH 7,25) wurden in einem Fermenter sterilisiert und nach Abkühlen mit 0,5 l der Vorkultur DSM 7063 angeimpft. Die Kulturbedingungen waren: 27°C, 300 Upm, 4 l Luft/min. Gleich nach dem Animpfen wurden 1000 ppm Eugenol zugegeben, sowie nach 6,5 Stunden 500 ppm und nach 8,5; 13,5; 19; 23,5; 26,5; 30,5; 37,5; und 45,5 Stunden nochmals je 1000 ppm. Nach 30,5 und 50 Stunden wurden zusätzlich je 2 g Caseinhydrolysat zugegeben.

Nach 75 Stunden wurde die Fermentation abgebrochen. Die Konzentration an Ferulasäure lag bei 5,8 g/l entsprechend einer Umsetzung von 51,6 % der Theorie.

Bei Fermentation von DSM 7062 bei 32°C lag die Konzentration an Ferulasäure nach 78 Stunden bei 6,7 g/l; dies sind bei Einsatz von 13,9 g/l Eugenol 41 % der Theorie.

## Patentansprüche

1. Pseudomonas sp. nov. mit den bei der Deutschen Sammlung für Mikroorganismen und Zellkulturen GmbH in Braunschweig unter den Nummern DSM 7062 und DSM 7063 hinterlegten Stämmen.

2. Verfahren zur Herstellung von Verbindungen der Formel 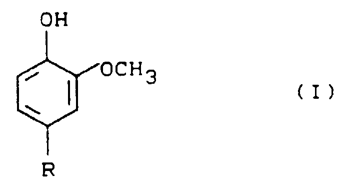 worin
R -COOH, -CH=CH-COOH, -CH=CH-CH₂OH oder -CH=CH-CHO bedeutet,
aus Eugenol 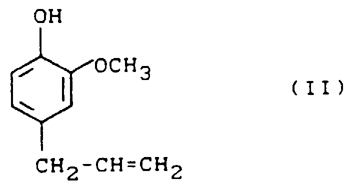 in Gegenwart von Pseudomonas sp. nach Anspruch 1 oder deren Enzymen oder von Mikrorganismen mit genetischem Material aus Pseudomonas nach Anspruch 1, das die Struktur- und Regulatorgene für die Enzyme kodiert, die in dieser Reaktion wirksam sind.

## Claims

1. Novel Pseudomonas sp. with the strains deposited at the Deutschen Sammlung für Mikroogranismen und Zellkulturen GmbH in Braunschweig under the numbers DSM 7062 and DSM 7063.

2. A process for preparing compounds of the formula 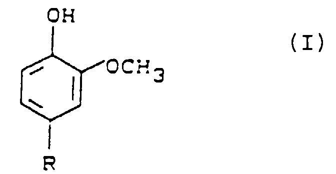 in which
R represents -COOH, -CH=CH-COOH, -CH=CH-CH₂OH or -CH=CH-CHO,
from eugenol 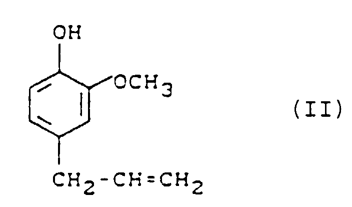 in the presence of Pseudomonas sp. according to Claim 1 or their enzymes or from microorganisms with genetic material from Pseudomonas according to Claim 1, which encode the structural and regulator genes for the enzymes which are active in this reaction.

## Revendications

1. Pseudomonas sp. nov. pour lequel des souches sont déposées auprès de « Deutchen Sammlung fur Mikroorganismen und Zellkulturen GmbH » à Braunschweig sous les numéros DSM 7062 et DSM 7063.

2. Procédé de préparation de composés de formule 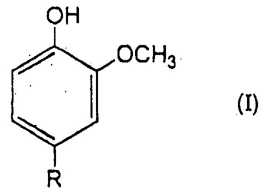 dans laquelle R signifie -COOH, -CH = CH-COOH, -CH = CH-CH₂OH ou -CH = CH-CHO,
à partir d'eugénol 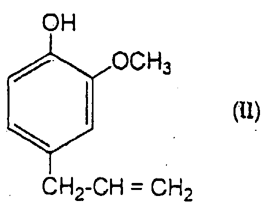 en présence de Pseumonas sp. nov. selon la revendication 1 ou ses enzymes ou de micro-organismes avec le matériel génétique de Pseumonas sp. nov. selon la revendication 1, qui code le gène de structure et régulateur pour les enzymes, qui sont actives dans cette réaction.
